(19) Europäisches Patentamt
European Patent Office
Office européen des brevets

(11) Veröffentlichungsnummer : **0 144 895**
**B1**

(12) # EUROPÄISCHE PATENTSCHRIFT

(45) Veröffentlichungstag der Patentschrift : **20.01.88**

(51) Int. Cl.[4] : **C 07 D333/38**, C 07 D409/12, A 01 N 43/10

(21) Anmeldenummer : **84114309.2**

(22) Anmeldetag : **27.11.84**

(54) **Thiolan-2,4-dion-3-carboxamide.**

(30) Priorität : **09.12.83 DE 3344523**
**27.07.84 DE 3427847**

(43) Veröffentlichungstag der Anmeldung : **19.06.85 Patentblatt 85/25**

(45) Bekanntmachung des Hinweises auf die Patenterteilung : **20.01.88 Patentblatt 88/03**

(84) Benannte Vertragsstaaten :
**AT BE CH DE FR GB IT LI NL SE**

(56) Entgegenhaltungen :
**US-A- 3 833 610**
**CHEMICAL ABSTRACTS, Band 87, Nr. 11, 12. September 1977, Seiten 580-581, Nr. 84822b, Columbus, Ohio, US; & JP - A - 77 46 078 (NIPPON SODA CO., LTD.) 12.04.1977**
**Die Akte enthält technische Angaben, die nach dem Eingang der Anmeldung eingereicht wurden und die nicht in dieser Patentschrift enthalten sind.**

(73) Patentinhaber : **BAYER AG**
**Konzernverwaltung RP Patentabteilung**
**D-5090 Leverkusen 1 Bayerwerk (DE)**

(72) Erfinder : **Gayer, Herbert, Dr.**
**Alfred-Delp-Strasse 4**
**D-4019 Monheim (DE)**
Erfinder : **Brandes, Wilhelm, Dr.**
**Eichendorffstrasse 3**
**D-5653 Leichlingen (DE)**
Erfinder : **Hänssler, Gerd, Dr.**
**Heymannstrasse 40**
**D-5090 Leverkusen 1 (DE)**
Erfinder : **Reinecke, Paul, Dr.**
**Lessingstrasse 11**
**D-5090 Leverkusen 3 (DE)**

**EP 0 144 895 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

**Beschreibung**

Die Erfindung betrifft neue Thiolan-2,4-dion-3-carboxamide, ein Verfahren zur ihrer Herstellung und ihre Verwendung als Schädlingsbekämpfungsmittel.

Es ist bereits bekannt, daß einige als Insektizide bekannte Tetrahydrothiopyran-3,5-dion-4-carboxamide, wie beispielsweise das Tetrahydrothiopyran-3,5-dion-4-[N-(4-chlorphenyl)]-carboxamid oder das Tetrahydrothiopyran-3,5-dion-4-[N-(3,4-dichlorphenyl)]-carboxamid, fungizide Nebenwirkungen besitzen (vgl. Jap. Pat. 77 46 078).

Die Wirkung dieser Verbindungen ist jedoch, insbesondere bei niedrigen Aufwandmengen und -konzentrationen, nicht immer völlig zufriedenstellend.

Es wurden neue Thiolan-2,4-dion-3-carboxamide der allgemeinen Formel (I),

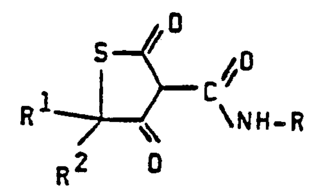

(I)

in welcher

R für geradkettiges oder verzweigtes Alkyl mit 1 bis 8 Kohlenstoffatomen, für Cycloalkyl mit 3 bis 7 Kohlenstoffatomen, für gegebenenfalls einfach oder mehrfach, gleich oder verschieden substituiertes Aralkyl mit 1 oder 2 Kohlenstoffatomen im Alkylteil und 6 bis 10 Kohlenstoffatomen im Arylteil, sowie für gegebenenfalls einfach oder mehrfach, gleich oder verschieden substituiertes Aryl mit 6 bis 10 Kohlenstoffatomen steht, wobei als Arylsubstituenten jeweils in Frage kommen : Halogen, Cyano, Nitro, geradkettiges oder verzweigtes Alkyl mit 1 bis 16 Kohlenstoffatomen, geradkettiges oder verzweigtes Alkoxy und Alkylthio mit jeweils 1 bis 4 Kohlenstoffatomen, Halogenalkyl, Halogenalkoxy und Halogenalkylthio mit jeweils 1 oder 2 Kohlenstoffatomen und bis zu 5 gleichen oder verschiedenen Halogenatomen, gegebenenfalls durch Halogen oder niederes Alkyl einfach oder mehrfach, gleich oder verschieden substituiertes zweifach verknüpftes Dioxyalkylen mit 1 oder 2 Kohlenstoffatomen im Alkylenrest, jeweils gegebenenfalls einfach oder mehrfach, gleich oder verschieden durch Halogen, niederes Alkyl und niederes Halogenalkyl substituiertes Phenyl oder Phenoxy, ferner Cycloalkyl mit 5 bis 7 Kohlenstoffatomen sowie Alkoxycarbonylalkyl und Alkoxycarbonylalkenyl mit jeweils 1 oder 2 Kohlenstoffatomen im Alkoxyteil und jeweils bis zu 2 Kohlenstoffatomen im Alkyl- bzw. Alkenylteil und

$R^1$ und $R^2$ unabhängig voneinander für Wasserstoff, für geradkettiges oder verzweigtes Alkyl mit 1 bis 6 Kohlenstoffatomen, für Cycloalkyl mit 3 bis 7 Kohlenstoffatomen, für Cycloalkylalkyl mit 3 bis 7 Kohlenstoffatomen im Cycloalkylteil und 1 oder 2 Kohlenstoffatomen im Alkylteil, für Phenylalkyl mit 1 oder 2 Kohlenstoffatomen im Alkylteil oder für Phenyl stehen,

gefunden.

Die Verbindungen der Formel (I) liegen im tautomeren Gleichgewicht vor mit Verbindungen der Formeln (Ia), (Ib) und (Ic),

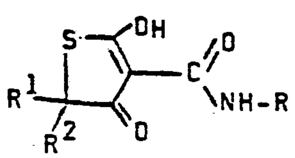

(Ia)

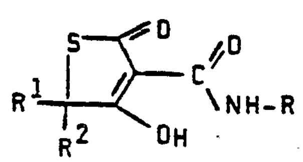

(Ib)

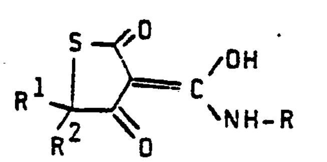

(Ic)

Besonders die Enolformen (Ia), (Ib) und (Ic) werden durch starke intramolekulare Wasserstoffbrücken stabilisiert.

Weiterhin können die Verbindungen der Formel (I) als geometrische und/oder optische Isomere oder Isomerengemische unterschiedlicher Zusammensetzung vorliegen.

Sowohl die reinen Isomeren als auch die Isomerengemische, ebenso wie die verschiedenen tautomeren Strukturen werden erfindungsgemäß beansprucht.

Weiterhin wurde gefunden, daß man die neuen Thiolan-2,4-dion-3-carboxamide der allgemeinen Formel (I),

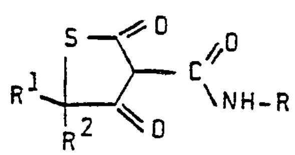

(I)

in welcher R, $R^1$ und $R^2$ die oben angegebenen Bedeutungen haben, erhält, wenn man Thiolan-2,4-dion-3-carbonsäureester der Formel (II),

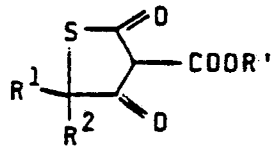

(II)

in welcher

$R^1$ und $R^2$ die oben angegebenen Bedeutungen haben und

R' für Alkyl steht,

mit Aminen der Formel (III),

$$H_2N—R \quad (III)$$

in welcher R die oben angegebenen Bedeutungen hat, gegebenenfalls in Gegenwart eines Verdünnungsmittels umsetzt.

Schließlich wurde gefunden, daß die neuen Thiolan-2,4-dion-3-carboxamide fungizide Eigenschaften besitzen.

Überraschenderweise zeigen die erfindungsgemäßen Thiolan-2,4-dion-3-carboxamide der Formel (I) eine bessere fungizide Wirksamkeit als die aus dem Stand der Technik bekannten Tetrahydrothiopyran-3,5-dion-4-carboxamide, wie beispielsweise das Tetrahydrothiopyran-3,5-dion-4-[N-(4-chlorphenyl)]-carboxamid bzw. -4-[N-(3,4-dichlorphenyl)]-carboxamid, welche chemisch und wirkungsmäßig sehr naheliegende Verbindungen sind.

Die erfindungsgemäßen Thiolan-2,4-dion-3-carboxamide sind durch die Formel (I) allgemein definiert. Bevorzugt sind Verbindungen der Formel (I), bei welchen

R für geradkettiges oder verzweigtes Alkyl mit 1 bis 6 Kohlenstoffatomen, für Cyclopentyl, für Cyclohexyl oder für gegebenenfalls einfach bis dreifach, gleich oder verschieden substituiertes Phenyl, Benzyl oder Naphthyl steht, wobei als Substituenten jeweils in Frage kommen : Fluor, Chlor, Brom, Nitro, Cyano, geradkettiges oder verzweigtes Alkyl mit 1 bis 14 Kohlenstoffatomen, Methoxy, Ethoxy, Methylthio, Trifluormethyl, Trifluormethoxy, Trifluormethylthio, jeweils zweifach verknüpftes Dioxymethylen, Dioxyethylen oder Dioxytrifluorchlorethylen, jeweils gegebenenfalls einfach bis dreifach, gleich oder verschieden durch Methyl, Chlor oder Trifluormethyl substituiertes Phenyl oder Phenoxy, Cyclopentyl oder Cyclohexyl, 2-Methoxycarbonylvinyl und 2-Ethoxycarbonylvinyl, und

$R^1$ und $R^2$ unabhängig voneinander für Wasserstoff, Methyl, Ethyl, n- und i-Propyl, n-, i-, s- und t-Butyl, Cyclohexyl, Cyclohexylmethyl, Benzyl oder Phenyl stehen.

Im einzelnen seien außer den bei den Herstellungsbeispielen genannten Verbindungen die folgenden Verbindungen der allgemeinen Formel (I) genannt :

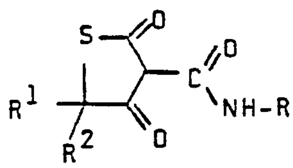

(I)

| R | $R^1$ | $R^2$ | R | $R^1$ | $R^2$ |
|---|---|---|---|---|---|
| 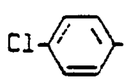 | $CH_3$ | $CH_3$ | 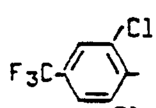 | H | H |
| 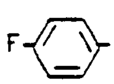 | $CH_3$ | $CH_3$ | 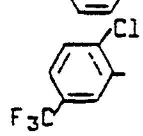 | H | H |
| 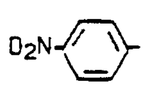 | $CH_3$ | $CH_3$ | 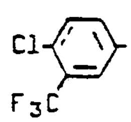 | H | H |
| 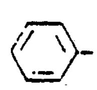 | $CH_3$ | $CH_3$ | | | |
| 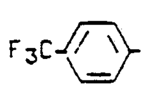 | H | H | 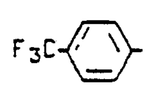 | H | H |

(Fortsetzung)

| R | $R^1$ | $R^2$ | R | $R^1$ | $R^2$ |
|---|---|---|---|---|---|
| 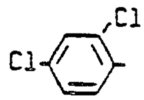 | H | H | 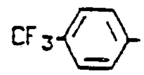 | H | $CH_3$ |
| 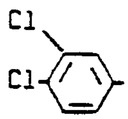 | $CH_3$ | $CH_3$ | 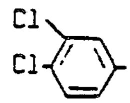 | $C_2H_5$ | $CH_3$ |
| 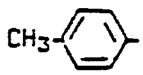 | $CH_3$ | $CH_3$ | 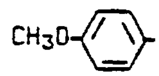 | $CH_3$ | $CH_3$ |
| 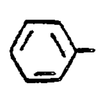 | $C_2H_5$ | $CH_3$ | 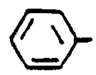 | $C_2H_5$ | H |
| 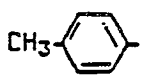 | $C_2H_5$ | $CH_3$ | 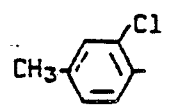 | $C_2H_5$ | H |
| 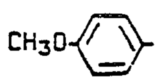 | $C_2H_5$ | $CH_3$ | 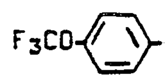 | H | $CH_3$ |
| 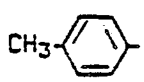 | $C_2H_5$ | H | 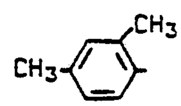 | H | $CH_3$ |

Verwendet man beispielsweise 5-Methylthiolan-2,4-dion-3-carbonsäureethylester und 4-Bromanilin als Ausgangsstoffe, so läßt sich der Reaktionsablauf des erfindungsgemäßen Verfahrens durch das folgende Formelschema darstellen :

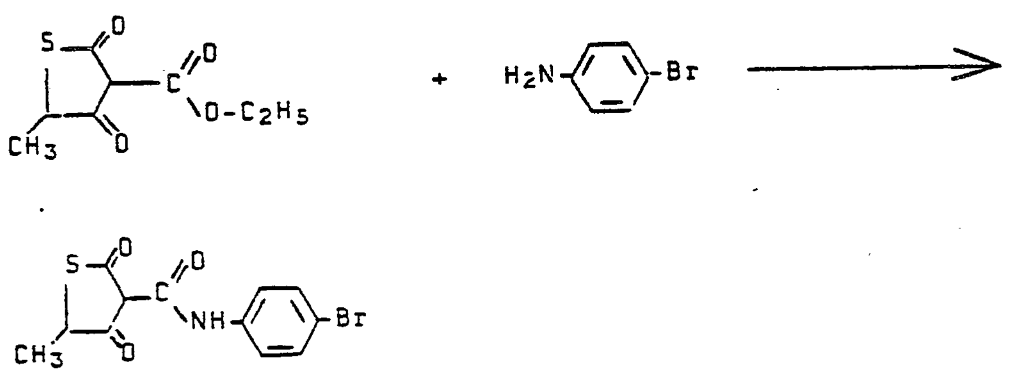

Die zur Durchführung des erfindungsgemäßen Verfahrens als Ausgangsstoffe benötigten Thiolan-2,4-dion-3-carbonsäureester sind durch die Formel (II) allgemein definiert. In dieser Formel (II) stehen $R^1$ und $R^2$ vorzugsweise für diejenigen Reste, die schon bei der Beschreibung der erfindungsgemäßen Stoffe der Formel (I) als bevorzugt für diese Substituenten genannt wurden ; R' steht vorzugsweise für geradkettiges oder verzweigtes Alkyl mit 1 bis 4 Kohlenstoffatomen, insbesondere für Methyl oder Ethyl.

Die Thiolan-2,4-dion-3-carbonsäureester der Formel (II) sind teilweise bekannt (vgl. z. B. Chem. Ber. 46, 2103 (1913)). Die noch nicht bekannten Vertreter erhält man in analoger Weise nach prinzipiell bekannten Verfahren, indem man zunächst in der 1. Stufe α-Halogencarbonsäuren der Formel (IV),

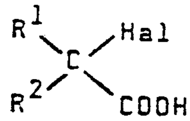

(IV)

in welcher

$R^1$ und $R^2$ die oben angegebenen Bedeutungen haben und

Hal für Halogen, vorzugsweise für Chlor oder Brom, steht,

mit Mercaptoessigsäure oder deren Natriumsalz, gegebenenfalls in Gegenwart eines Verdünnungsmittels, wie beispielsweise Tetrahydrofuran oder Wasser, und gegebenenfalls in Gegenwart eines Säurebindemittels, wie beispielsweise Triethylamin, bei Temperaturen zwischen — 20 °C und + 50 °C umsetzt, und die so erhältlichen α-Acetylthiocarbonsäuren der Formel (V),

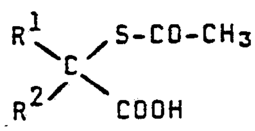

(V)

in welcher $R^1$ und $R^2$ die oben angegebenen Bedeutungen haben, in einer 2. Stufe in allgemein üblicher Weise mit einem Halogenierungsmittel, wie z. B. Thionylchlorid oder Oxalylchlorid, gegebenenfalls in Gegenwart eines Verdünnungsmittels, wie beispielsweise Dichlormethan, bei Temperaturen zwischen — 20 °C und + 50 °C halogeniert (vgl. z. B. J. Chem. Soc. C, 1968, 1504), und die so erhältlichen α-Acetylthiocarbonsäurehalogenide der Formel (VI),

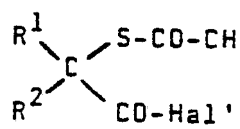

(VI)

in welcher

$R^1$ und $R^2$ die oben angegebenen Bedeutungen haben und

Hal' für Halogen, insbesondere Chlor oder Brom, steht,

in einer 3. Stufe mit dem Natriumsalz oder Ethoxymagnesiumsalz eines Malonsäureesters der Formel (VII),

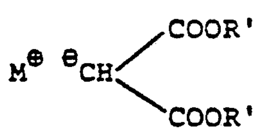

(VII)

in welcher

R' die oben angegebene Bedeutung hat und

$M^{\oplus}$ für ein Natrium- oder Ethoxymagnesiumkation steht,

gegebenenfalls in Gegenwart eines Verdünnungsmittels, wie beispielsweise Tetrahydrofuran, bei Temperaturen zwischen — 20 °C und + 50 °C umsetzt und das so erhältliche Malonesteraddukt der Formel (VIII),

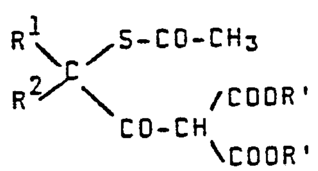

(VIII)

in welcher $R^1$, $R^2$ und R' die oben angegebenen Bedeutungen haben, in einer 4. Stufe gegebenenfalls in Gegenwart eines Verdünnungsmittels, wie beispielsweise Wasser, und gegebenenfalls in Gegenwart einer Base, wie beispielsweise Natriumhydroxid, bei Temperaturen zwischen 0 °C und 80 °C cyclisiert.

Malonsäureester-Natriumsalze der Formel (VIII), Mercaptoessigsäure und α-Halogencarbonsäuren der Formel (IV) sind allgemein bekannte Verbindungen der organischen Chemie.

Die weiterhin zur Durchführung des erfindungsgemäßen Verfahrens als Ausgangsstoffe benötigten Amine sind durch die Formel (III) allgemein definiert. In dieser Formel (III) steht R vorzugsweise für diejenigen Reste, die schon bei der Beschreibung der erfindungsgemäßen Stoffe der Formel (I) als bevorzugt für diesen Substituenten genannt wurden.

Die Amine der Formel (III) sind allgemein bekannte Verbindungen der organischen Chemie.

Das erfindungsgemäße Verfahren kann gegebenenfalls in Gegenwart eines Verdünnungsmittels durchgeführt werden. Als solche kommen prinzipiell alle inerten organischen Lösungsmittel in Frage. Bevorzugt verwendet man aromatische Kohlenwasserstoffe, wie Benzol, Toluol, Xylol ; Ether, wie Dioxan, Tetrahydrofuran oder Ethylenglykol-dimethylether bzw. -diethylether oder auch dipolar-aprotische Lösungsmittel, wie Dimethylformamid, Dimethylsulfoxid oder Hexamethylphosphorsäuretriamid.

Das erfindungsgemäße Verfahren kann jedoch auch in Abwesenheit eines Verdünnungsmittels

durchgeführt werden.

Die Reaktionstemperaturen können bei der Durchführung des erfindungsgemäßen Verfahrens in einem größeren Bereich variiert werden. Im allgemeinen arbeitet man zwischen + 50 °C und + 200 °C, vorzugsweise zwischen + 80 °C und + 150 °C.

Zur Durchführung des erfindungsgemäßen Verfahrens setzt man pro Mol Thiolan-2,4-dion-3-carbonsäureester der Formel (II) im allgemeinen 1 bis 2 Mol, vorzugsweise äquimolare Mengen I, an Amin der Formel (III) ein. Die Reaktionspartner werden mit oder ohne Lösungsmittel für einige Minuten bis einige Stunden auf die erforderliche Reaktionstemperatur erhitzt. Beim Abkühlen des Reaktionsgemisches erhält man die gewünschten Endprodukte der Formel (I) in der Regel in kristalliner Form, welche sich durch Umkristallisieren aus einem geeigneten Lösungsmittel reinigen lassen.

Die erfindungsgemäßen Wirkstoffe weisen eine starke mikrobizide Wirkung auf und können zur Bekämpfung von unerwünschten Mikroorganismen praktisch eingesetzt werden. Die Wirkstoffe sind für den Gebrauch als Pflanzenschutzmittel geeignet.

Fungizide Mittel im Pflanzenschutz werden eingesetzt zur Bekämpfung von Plasmodiophoromycetes, Oomycetes, Chytridiomycetes, Zygomycetes, Ascomycetes, Basidiomycetes, Deuteromycetes.

Bakterizide Mittel werden im Pflanzenschutz zur Bekämpfung von Pseudomonadaceae, Rhizobiaceae, Enterobacteriaceae, Corynebacteriaceae und Streptomycetaceae eingesetzt.

Die gute Pflanzenverträglichkeit der Wirkstoffe in den zur Bekämpfung von Pflanzenkrankheiten notwendigen Konzentrationen erlaubt eine Behandlung von oberirdischen Pflanzenteilen, von Pflanz- und Saatgut, und des Bodens.

Die erfindungsgemäßen Wirkstoffe lassen sich mit besonders gutem Erfolg zur Bekämpfung von Getreidekrankheiten, z. B. verursacht durch Cochliobolus sativus, Septoria nodorum, Pyrenophora teres und Puccinia-Arten, zur Bekämpfung von Reiskrankheiten, wie beispielsweise gegen den Erreger der Reisfleckenkrankheit (Pyricularia oryzae) oder gegen den Erreger der Reiskrankheit (Pellicularia sasakii), oder zur Bekämpfung von Obst- und Gemüsekrankheiten, wie beispielsweise gegen den Erreger der Tomatenkrautfäule (Phytophthora infestans) oder gegen den Erreger des falschen Rebenmehltaus (Plasmopara viticola) einsetzen.

Daneben besitzen die erfindungsgemäßen Wirkstoffe auch eine bakterizide, insektizide und insektenentwicklungshemmende Wirkung.

Die Wirkstoffe können in die üblichen Formulierungen übergeführt werden, wie Lösungen, Emulsionen, Spritzpulver, Suspensionen, Pulver, Stäubemittel, Schäume, Pasten, lösliche Pulver, Granulate, Aerosole, Suspensions-Emulsionkonzentrate, Saatgutpuder, Wirkstoff-imprägnierte Natur- und synthetische Stoffe, Feinstverkapselungen in polymeren Stoffen und in Hüllmassen für Saatgut, ferner in Formulierungen mit Brennsätzen, wie Räucherpatronen, -dosen, -spiralen u. ä., sowie ULV-Kalt- und Warmnebel-Formulierungen.

Diese Formulierungen werden in bekannter Weise hergestellt, z. B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z. B. auch organische Lösungsmittel als Hilfslösungsmittel verwendet werden. Als flüssige Lösungsmittel kommen im wesentlichen in Frage : Aromaten, wie Xylol, Toluol, oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z. B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser ; mit verflüssigten gasförmigen Streckmitteln oder Trägerstoffen sind solche Flüssigkeiten gemeint, welche bei normaler Temperatur und unter Normaldruck gasförmig sind, z. B. Aerosol-Treibgase, wie Halogenkohlenwasserstoffe sowie Butan, Propan, Stickstoff und Kohlendioxid ; als feste Trägerstoffe kommen in Frage : z. B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate ; als feste Trägerstoffe für Granulate kommen in Frage : z. B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel ; als Emulgier- und/oder schaumerzeugende Mittel kommen in Frage : z. B. nichtionogene und anionische Emulgatoren, wie Polyoxyethylen-Fettsäure-Ester, Polyoxyethylen-Fettalkohol-Ether, z. B. Alkylarylpolyglykol-Ether, Alkylsulfonate, Alkylsulfate, Arylsulfonate sowie Eiweißhydrolysate ; als Dispergiermittel kommen in Frage : z. B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat, sowie natürliche Phospholipide, wie Kephaline und Lecithine, und synthetische Phospholipide. Weitere Additive können mineralische und vegetabile Öle sein.

Es können Farbstoffe wie anorganische Pigmente, z. B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können in den Formulierungen oder in den verschiedenen Anwendungsformen in Mischung mit anderen bekannten Wirkstoffen vorliegen, wie Fungiziden, Bakteriziden, Insektiziden, Akariziden, Nematiziden, Herbiziden, Schutzstoffen gegen Vogelfraß, Wuchsstoffen, Pflanzennährstoffen und Bodenstrukturverbesserungsmitteln.

Die Wirkstoffe können als solche, in Form ihrer Formulierungen oder der daraus durch weiteres Verdünnen bereiteten Anwendungsformen, wie gebrauchsfertige Lösungen, Emulsionen, Suspensionen, Pulver, Pasten und Granulate angewendet werden. Die Anwendung geschieht in üblicher Weise, z. B. durch Gießen, Tauchen, Spritzen, Sprühen, Vernebeln, Verdampfen, Injizieren, Verschlämmen, Verstreichen, Stäuben, Streuen, Trockenbeizen, Feuchtbeizen, Naßbeizen, Schlämmbeizen oder Inkrustieren.

Bei der Behandlung von Pflanzenteilen können die Wirkstoffkonzentrationen in den Anwendungsformen in einem größeren Bereich variiert werden. Sie liegen im allgemeinen zwischen 1 und 0,000 1 Gew.-%, vorzugsweise zwischen 0,5 und 0,001 %.

Bei der Saatgutbehandlung werden im allgemeinen Wirkstoffmengen von 0,001 bis 50 g je Kilogramm Saatgut, vorzugsweise 0,1 bis 10 g, benötigt.

Bei Behandlung des Bodens sind Wirkstoffkonzentrationen von 0,000 01 bis 0,1 Gew.-%, vorzugsweise von 0,000 1 bis 0,02 Gew.-%, am Wirkungsort erforderlich.

## Herstellungsbeispiele

### Beispiel 1

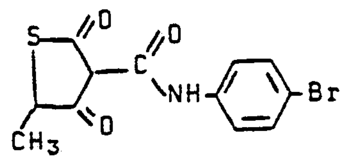

5,1 g (0,025 Mol) 5-Methylthiolan-2,4-dion-3-carbonsäureethylester und 4,3 g (0,025 Mol) 4-Bromanilin werden zusammen in 25 ml Toluol eine Stunde am Rückfluß gekocht. Nach beendeter Reaktion wird die Mischung auf 0 °C abgekühlt, wobei ein Teil des Produktes auskristallisiert. Eine weitere Fraktion erhält man durch Einengen der Mutterlauge und Umkristallisation des Rückstandes aus 30 ml Ethanol.

Insgesamt erhält man 7,4 g (90 % der Theorie) an 5-Methylthiolan-2,4-dion-3-[N-(4-bromphenyl)]-carboxamid vom Schmelzpunkt 132 °C bis 134 °C.

Herstellung der Ausgangsverbindung

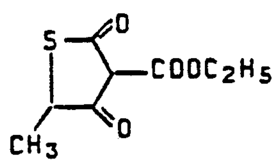

14,4 g (0,05 Mol) 2-Acetylthiopropionylmalonsäurediethylester werden in 50 ml (0,1 Mol) 2N-Natronlauge eingetragen. Man läßt die sich bildende Lösung 30 Minuten bei Raumtemperatur stehen, säuert dann an und extrahiert mit Essigester. Die vereinigten Essigesterphasen werden über Natriumsulfat getrocknet und im Vakuum eingeengt. Das kristalline Rohprodukt läßt sich aus Ethanol umkristallisieren.

Man erhält 8,6 g (85 % der Theorie) an 5-Methylthiolan-2,4-dion-3-carbonsäureethylester vom Schmelzpunkt 82 °C bis 83 °C.

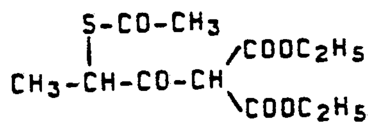

19,2 g (0,12 Mol) Malonsäurediethylester werden in eine Lösung von 6,5 g (0,12 Mol) Natriummethylat in 100 ml Ethanol gegeben. Man entfernt das Lösungsmittel im Vakuum und löst den Rückstand in 100 ml Tetrahydrofuran. Zu der so erhaltenen Lösung tropft man bei 0 °C unter Rühren 10 g (0,06 Mol) 2-Acetylthiopropionsäurechlorid und rührt nach beendeter Zugabe eine Stunde bei Raumtemperatur. Zur Aufarbeitung entfernt man das Lösungsmittel im Vakuum, verteilt den Rückstand in einem Essigester-Wasser-Gemisch, trennt die organische Phase ab und entfernt alle flüchtigen Bestandteile im Hochvakuum. Als Rückstand verbleiben 14,4 g (82,6 % der Theorie) an rohem 2-Acetylthiopropionylmalonsäurediethylester, der für weitere Umsetzungen ohne weitere Reinigung verwendet werden kann.

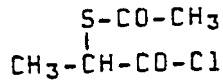

44,3 g (0,3 Mol) 2-Acetylthiopropionsäure in 460 ml trockenem Dichlormethan werden bei Raumtemperatur tropfenweise mit 66,2 g (0,52 Mol) Oxalylchlorid versetzt und nach beendeter Zugabe bis zum Ende der Gasentwicklung bei Raumtemperatur gerührt.

Zur Aufarbeitung entfernt man Lösungsmittel und überschüssiges Oxalylchlorid im Vakuum und destilliert den Rückstand im Hochvakuum.

Man erhält 22,7 g (45 % der Theorie) an 2-Acetylthiopropionsäurechlorid vom Siedepunkt 60 °C bei 1,3 mbar.

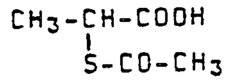

45,9 g (0,3 Mol) 2-Brompropionsäure in 300 ml Tetrahydrofuran werden bei 0 °C tropfenweise unter Rühren mit 60,7 g (0,6 Mol) Triethylamin und anschließend mit 22,8 g (0,3 Mol) Thioessigsäure versetzt. Nach beendeter Zugabe rührt man weitere 3 Stunden bei 0 °C und hierauf 12 Stunden bei Raumtemperatur. Zur Aufarbeitung entfernt man das Lösungsmittel im Vakuum und verteilt den Rückstand in einem Gemisch aus 300 ml Essigester und 300 ml Wasser. Die organische Phase wird abgetrennt, über Natriumsulfat getrocknet und im Vakuum vom Lösungsmittel befreit.

Man erhält 44,5 g (100 % der Theorie) an 2-Acetylthiopropionsäure, die ohne weitere Reinigung verwendet werden kann.

In entsprechender Weise und gemäß den allgemeinen Herstellungsangaben erhält man die folgenden Verbindungen der allgemeinen Formel (I) :

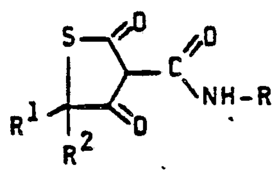

(I)

| Beisp. Nr. | R | $R^1$ | $R^2$ | Schmelzpunkt [°C] |
|---|---|---|---|---|
| 2 | 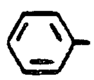 | H | H | 158 |
| 3 | 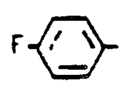 | H | H | 169 |
| 4 | 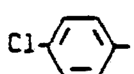 | H | H | 214 |
| 5 | 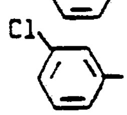 | H | H | 183 |
| 6 | 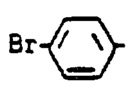 | H | H | 213 |
| 7 | 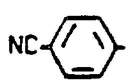 | H | H | > 220 |
| 8 | 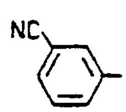 | H | H | 230 |
| 9 | 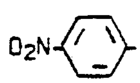 | H | H | 265 |

(Fortsetzung)

| Beisp. Nr. | R | $R^1$ | $R^2$ | Schmelzpunkt [°C] |
|---|---|---|---|---|
| 10 | 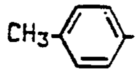 | H | H | 173 |
| 11 | 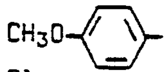 | H | H | 145 |
| 12 | 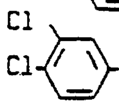 | H | H | 216 |
| 13 | 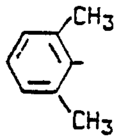 | H | H | 133 |
| 14 | 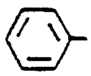 | $CH_3$ | H | 102 |
| 15 | 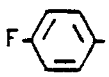 | $CH_3$ | H | 127 |
| 16 | 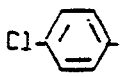 | $CH_3$ | H | 136 |
| 17 | 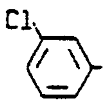 | $CH_3$ | H | 124 |
| 18 | 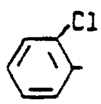 | $CH_3$ | H | 107 |
| 19 | 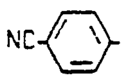 | $CH_3$ | H | 208 |
| 20 | 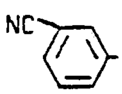 | $CH_3$ | H | 157 |
| 21 | 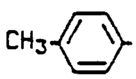 | $CH_3$ | H | 92 |
| 22 | 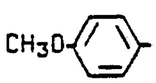 | $CH_3$ | H | 93,5 |
| 23 | 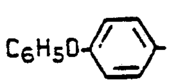 | $CH_3$ | H | 90 |
| 24 | 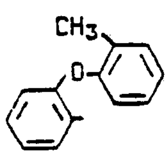 | $CH_3$ | H | 102 |
| 25 | 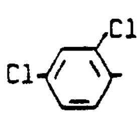 | $CH_3$ | H | 172 |

(Fortsetzung)

| Beisp. Nr. | R | $R^1$ | $R^2$ | Schmelzpunkt [°C] |
|---|---|---|---|---|
| 26 | 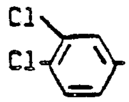 | $CH_3$ | H | 156 |
| 27 | 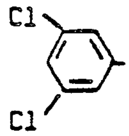 | $CH_3$ | H | 105 |
| 28 | 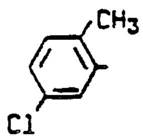 | $CH_3$ | H | 135 |
| 29 | 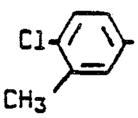 | $CH_3$ | H | 106 |
| 30 | 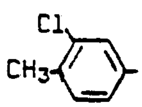 | $CH_3$ | H | 105 |
| 31 | 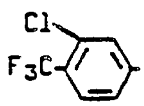 | $CH_3$ | H | 135 |
| 32 | 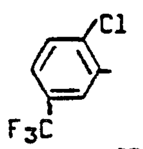 | $CH_3$ | H | 137 |
| 33 | 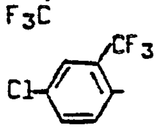 | $CH_3$ | H | 95 |
| 34 | 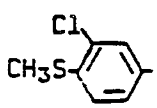 | $CH_3$ | H | 141 |
| 35 | 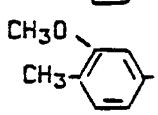 | $CH_3$ | H | 101 |
| 36 | 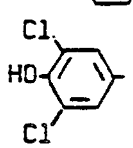 | $CH_3$ | H | 177 |
| 37 | 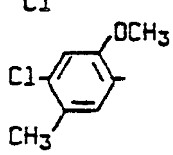 | $CH_3$ | H | 165 |
| 38 | 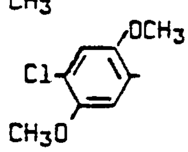 | $CH_3$ | H | 137 |

(Fortsetzung)

| Beisp. Nr. | R | $R^1$ | $R^2$ | Schmelzpunkt [°C] |
|---|---|---|---|---|
| 39 | 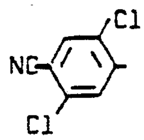 | $CH_3$ | H | 178 |
| 40 | 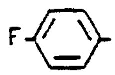 | $C_2H_5$ | H | 141 |
| 41 | 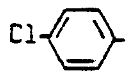 | $C_2H_5$ | H | 154 |
| 42 | 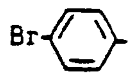 | $C_2H_5$ | H | 153 |
| 43 | 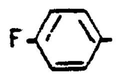 | $(CH_3)_2CH-$ | H | 140 |
| 44 | 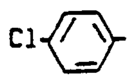 | $(CH_3)_2CH-$ | H | 161 |
| 45 | 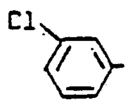 | $(CH_3)_2CH-$ | H | 137 |
| 46 | 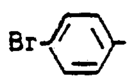 | $(CH_3)_2CH-$ | H | 163 |
| 47 | 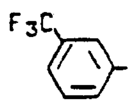 | $(CH_3)_2CH-$ | H | 107 |
| 48 | 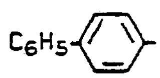 | $(CH_3)_2CH-$ | H | 112 |
| 49 | 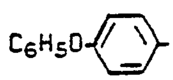 | $(CH_3)_2CH-$ | H | 106 |
| 50 | 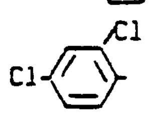 | $(CH_3)_2CH-$ | H | 132 |
| 51 | 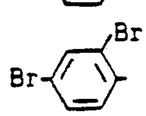 | $(CH_3)_2CH-$ | H | 122 |
| 52 | 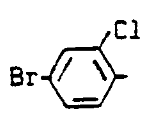 | $(CH_3)_2CH-$ | H | 128 |
| 53 | 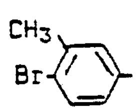 | $(CH_3)_2CH-$ | H | 149 |
| 54 | 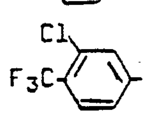 | $(CH_3)_2CH-$ | H | 112 |
| 55 | 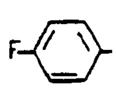 | $(CH_3)_3C-$ | H | 149 |

(Fortsetzung)

| Beisp. Nr. | R | $R^1$ | $R^2$ | Schmelzpunkt [°C] |
|---|---|---|---|---|
| 56 | 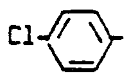 | $(CH_3)_3C-$ | H | 168 |
| 57 | 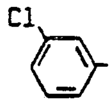 | $(CH_3)_3C-$ | H | 122 |
| 58 | 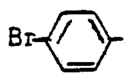 | $(CH_3)_3C-$ | H | 160 |
| 59 | 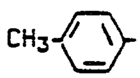 | $(CH_3)_3C-$ | H | 155 |
| 60 | 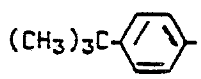 | $(CH_3)_3C-$ | H | 158 |
| 61 | 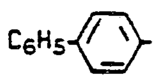 | $(CH_3)_3C-$ | H | 175 |
| 62 | 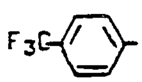 | $(CH_3)_3C-$ | H | 159 |
| 63 | 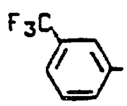 | $(CH_3)_3C-$ | H | 120 |
| 64 | 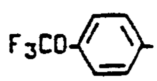 | $(CH_3)_3C-$ | H | 143 |
| 65 | 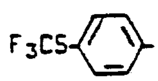 | $(CH_3)_3C-$ | H | 177 |
| 66 | 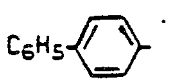 | $(CH_3)_3C-$ | H | 133 |
| 67 | 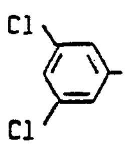 | $(CH_3)_3C-$ | H | 75 |
| 68 | 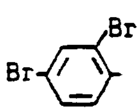 | $(CH_3)_3C-$ | H | 139 |
| 69 | 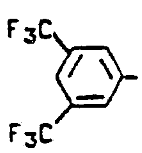 | $(CH_3)_3C-$ | H | 91 |
| 70 | 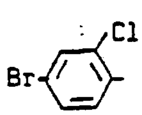 | $(CH_3)_3C-$ | H | 110 |
| 71 | 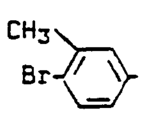 | $(CH_3)_3C-$ | H | 136 |

(Fortsetzung)

| Beisp. Nr. | R | $R^1$ | $R^2$ | Schmelzpunkt [°C] |
|---|---|---|---|---|
| 72 | 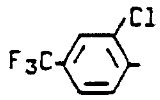 | $(CH_3)_3C-$ | H | 127 |
| 73 | 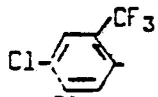 | $(CH_3)_3C-$ | H | 104 |
| 74 | 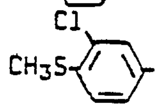 | $(CH_3)_3C-$ | H | 142 |
| 75 | 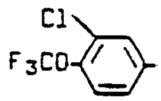 | $(CH_3)_3C-$ | H | 143 |
| 76 | 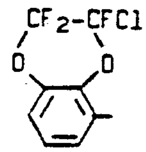 | $(CH_3)_3C-$ | H | 129 |
| 77 | 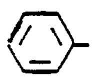 | $C_6H_5-$ | H | 175 |
| 78 | 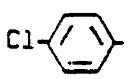 | $C_6H_5-$ | H | 228 |
| 79 | 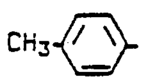 | $C_6H_5-$ | H | 181 |
| 80 | 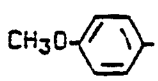 | $C_6H_5-$ | H | 180 |
| 81 | 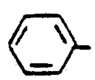 | 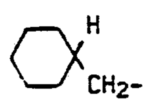 | H | 135 |
| 82 | 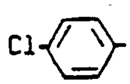 | 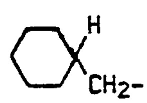 | H | 185 |
| 83 | 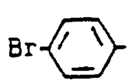 | 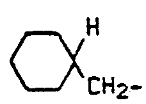 | H | 199-201 |
| 84 | 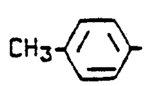 | 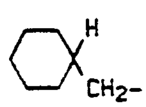 | H | 128-130 |
| 85 | 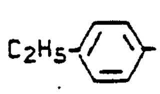 | 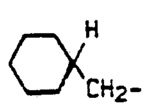 | H | 105-106 |
| 86 | 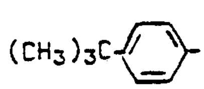 | 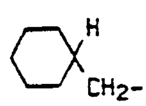 | H | 141-143 |
| 87 | 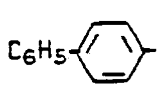 | 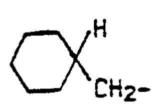 | H | 130 |

(Fortsetzung)

| Beisp. Nr. | R | $R^1$ | $R^2$ | Schmelzpunkt [°C] |
|---|---|---|---|---|
| 88 | 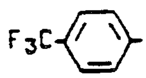 | 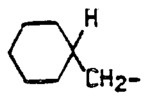 | H | 175 |
| 89 | 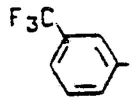 | 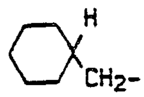 | H | 109-110 |
| 90 | 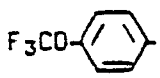 | 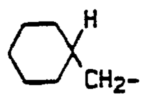 | H | 144-147 |
| 91 | 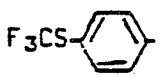 | 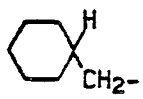 | H | 167-168 |
| 92 | 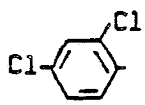 | 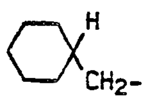 | H | 119-122 |
| 93 | 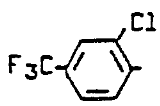 | 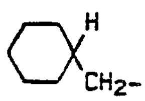 | H | 147 |
| 94 | 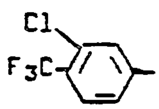 | 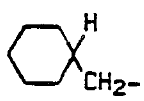 | H | 130-133 |
| 95 | 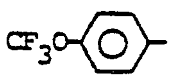 | $CH_3$ | H | 136 |
| 96 | 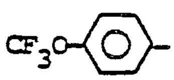 | H | H | 166 |
| 97 | 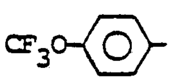 | $(CH_3)_2CH-$ | H | 135-37 |
| 98 | 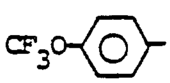 | $C_2H_5$ | H | 143-44 |
| 99 | 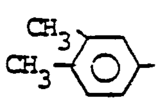 | $CH_3$ | H | 88-90 |
| 100 | 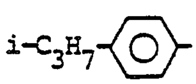 | $CH_3$ | H | 85-86 |
| 101 | 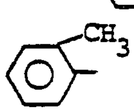 | $CH_3$ | H | 107-08 |
| 102 | 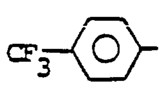 | $CH_3$ | H | 152-53 |
| 103 | 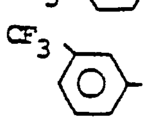 | $CH_3$ | H | 91 |

(Fortsetzung)

| Beisp. Nr. | R | $R^1$ | $R^2$ | Schmelzpunkt [°C] |
|---|---|---|---|---|
| 104 | 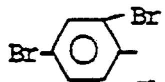 | $CH_3$ | H | 197-98 |
| 105 | 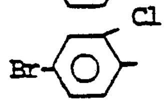 | $CH_3$ | H | 170-71 |
| 106 | 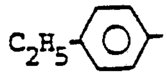 | $CH_3$ | H | 89-90 |
| 107 | 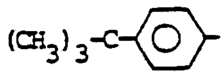 | $CH_3$ | H | 119 |
| 108 | 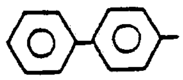 | $CH_3$ | H | 133-34 |
| 109 | 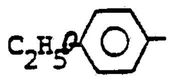 | $CH_3$ | H | Öl |
| 110 | 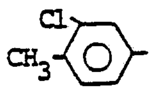 | $CH_3$ | H | Öl |
| 111 | 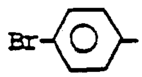 | $n-C_3H_7-$ | H | 142-44 |
| 112 | 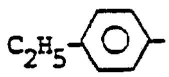 | $n-C_3H_7-$ | H | 102-03 |
| 113 | 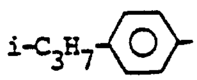 | $n-C_3H_7-$ | H | 97-98 |
| 114 | 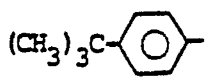 | $n-C_3H_7-$ | H | 80-83 |
| 115 | 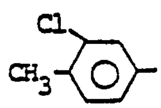 | $n-C_3H_7-$ | H | 96-97 |
| 116 | 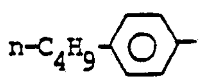 | $n-C_3H_7-$ | H | 94-95 |
| 117 | 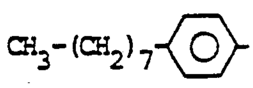 | $n-C_3H_7-$ | H | 82-83 |
| 118 | 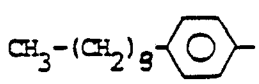 | $n-C_3H_7-$ | H | 87-88 |
| 119 | 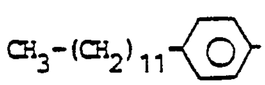 | $n-C_3H_7-$ | H | 72-74 |

(Fortsetzung)

| Beisp. Nr. | R | $R^1$ | $R^2$ | Schmelzpunkt [°C] |
|---|---|---|---|---|
| 120 | 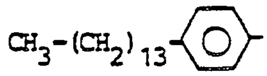 | $n-C_3H_7-$ | H | 73-74 |
| 121 | 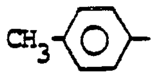 | $n-C_3H_7-$ | H | 104 |
| 122 | 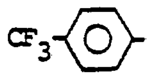 | $n-C_3H_7-$ | H | 156-58 |
| 123 | 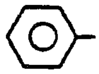 | $n-C_3H_7-$ | H | 105-06 |
| 124 | 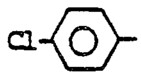 | $n-C_3H_7-$ | H | 133-34 |
| 125 | 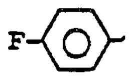 | $n-C_3H_7-$ | H | 131-33 |
| 126 | 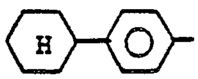 | $n-C_3H_7-$ | H | 129-30 |
| 127 | 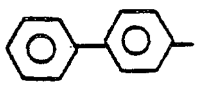 | $n-C_3H_7-$ | H | Öl |
| 128 | 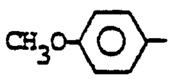 | $n-C_3H_7-$ | H | 114-15 |
| 129 | 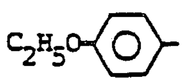 | $n-C_3H_7-$ | H | 108-09 |
| 130 | 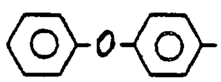 | $n-C_3H_7-$ | H | 109-11 |
| 131 | 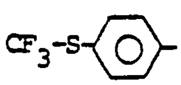 | $n-C_3H_7-$ | H | 177-79 |
| 132 | 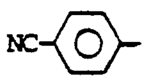 | $n-C_3H_7-$ | H | Öl |
| 133 | 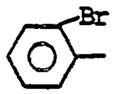 | $n-C_3H_7-$ | H | Öl |
| 134 | 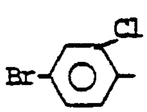 | $n-C_3H_7-$ | H | 123 |
| 135 | 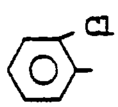 | $n-C_3H_7-$ | H | 90-92 |

(Fortsetzung)

| Beisp. Nr. | R | $R^1$ | $R^2$ | Schmelzpunkt [°C] |
|---|---|---|---|---|
| 136 | 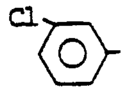 | $n-C_3H_7-$ | H | 133-34 |
| 137 | 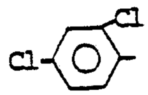 | $n-C_3H_7-$ | H | 112-13 |
| 138 | 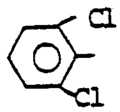 | $n-C_3H_7-$ | H | 124-27 |
| 139 | 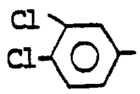 | $n-C_3H_7-$ | H | 128-29 |
| 140 | 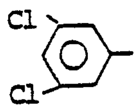 | $n-C_3H_7-$ | H | 154-56 |
| 141 | 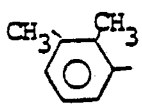 | $n-C_3H_7-$ | H | 94-96 |
| 142 | 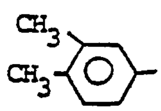 | $n-C_3H_7-$ | H | 76-78 |
| 143 | 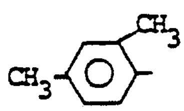 | $n-C_3H_7-$ | H | 95-96 |
| 144 | 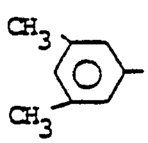 | $n-C_3H_7-$ | H | 100-01 |
| 145 | 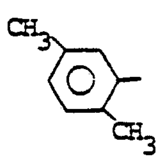 | $n-C_3H_7-$ | H | 103-04 |
| 146 | 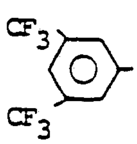 | $n-C_3H_7-$ | H | 93-95 |

(Fortsetzung)

| Beisp. Nr. | R | $R^1$ | $R^2$ | Schmelzpunkt [°C] |
|---|---|---|---|---|
| 147 | 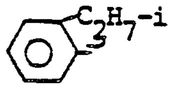 | n-$C_3H_7$- | H | Öl |
| 148 | 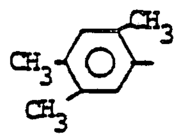 | n-$C_3H_7$- | H | 108–09 |
| 149 | 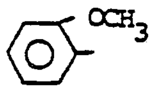 | n-$C_3H_7$- | H | 104–05 |
| 150 | 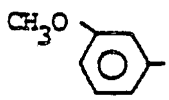 | n-$C_3H_7$- | H | 70–72 |
| 151 | 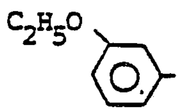 | n-$C_3H_7$- | H | 76–77 |
| 152 | 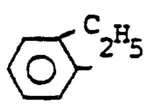 | n-$C_3H_7$- | H | 54–58 |
| 153 | 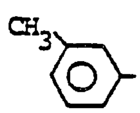 | n-$C_3H_7$- | H | 73–74 |
| 154 | 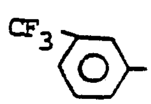 | n-$C_3H_7$- | H | 98–99 |
| 155 | 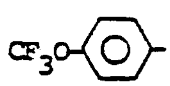 | n-$C_3H_7$- | H | 149–50 |
| 156 | 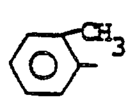 | n-$C_3H_7$- | H | 99–100 |
| 157 | 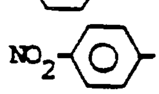 | n-$C_3H_7$- | H | 194–97 |
| 158 | 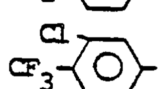 | n-$C_3H_7$- | H | 145–47 |
| 159 | 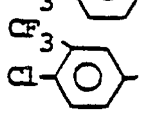 | n-$C_3H_7$- | H | 79–82 |

(Fortsetzung)

| Beisp. Nr. | R | $R^1$ | $R^2$ | Schmelzpunkt [°C] |
|---|---|---|---|---|
| 160 | 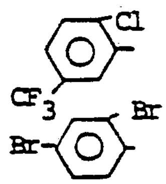 | $n-C_3H_7-$ | H | 100–01 |
| 161 | | $n-C_3H_7-$ | H | 129–31 |
| 162 | 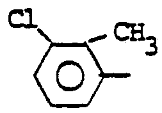 | $n-C_3H_7-$ | H | 105–06 |
| 163 | 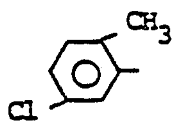 | $n-C_3H_7-$ | H | Öl |

Anwendungsbeispiele

In den folgenden Anwendungsbeispielen werden die nachstehend aufgeführten Verbindungen als Vergleichssubstanzen verwendet :

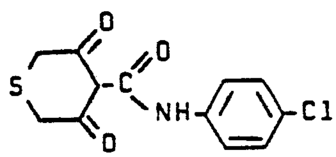

Tetrahydrothiopyran-3,5-dion-4-[N-(4-chlorphenyl)]-carboxamid

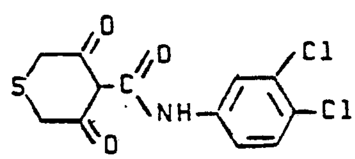

Tetrahydrothiopyran-3,5-dion-4-[N-(3,4-dichlorphenyl)]-carboxamid

(Beide bekannt aus Jap. Pat. 77 46 078 vom 12.4.1977)

Beispiel A

Pyricularia-Test (Reis)/protektiv

Lösungsmittel : 12,5 Gewichtsteile Aceton
Emulgator : 0,3 Gewichtsteile Alkylarylpolyglykolether

Zur Herstellung einer zweckmäßigen Wirkstoffzubereitung vermischt man 1 Gewichtsteil Wirkstoff mit der angegebenen Menge Lösungsmittel und verdünnt das Konzentrat mit Wasser und der angegebenen Menge Emulgator auf die gewünschte Konzentration.

Zur Prüfung auf protektive Wirksamkeit bespritzt man junge Reispflanzen mit der Wirkstoffzubereitung bis zur Tropfnässe. Nach dem Abtrocknen des Spritzbelages werden die Pflanzen mit einer wäßrigen Sporensuspension von Pyricularia oryzae inokuliert. Anschließend werden die Pflanzen in einem Gewächshaus bei 100 % rel. Luftfeuchtigkeit und 25 °C aufgestellt.

4 Tage nach der Inokulation erfolgt die Auswertung des Krankheitsbefalls.

Eine deutliche Überlegenheit in der Wirksamkeit gegenüber dem Stand der Technik zeigen in diesem Test z. B. die Verbindungen gemäß den Herstellungsbeispielen 3, 23, 26, 40, 70 und 71.

Beispiel B

Cochliobolus sativus-Test (Gerste)/protektiv

Lösungsmittel : 100 Gewichtsteile Dimethylformamid
Emulgator : 0,25 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit besprüht man junge Pflanzen mit der Wirkstoffzubereitung taufeucht. Man Antrocknen des Spritzbelages werden die Pflanzen mit einer Konidiensuspension von Cochliobolus sativus besprüht. Die Pflanzen verbleiben 48 Stunden bei 20 °C und 100 % rel. Luftfeuchtigkeit in einer Inkubationskabine.

Die Pflanzen werden in einem Gewächshaus bei einer Temperatur von ca. 20 °C und einer relativen Luftfeuchtigkeit von ca. 80 % aufgestellt.

7 Tage nach der Inokulation erfolgt die Auswertung.

Eine deutliche Überlegenheit in der Wirksamkeit gegenüber dem Stand der Technik zeigen bei diesem Test z. B. die Verbindungen gemäß den Herstellungsbeispielen 41 und 42.

Beispiel C

Phytophthora-Test (Tomate)/protektiv

Lösungsmittel : 4,7 Gewichsteile Aceton
Emulgator : 0,3 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit bespritzt man junge Pflanzen mit der Wirkstoffzubereitung bis zur Tropfnässe. Nach Antrocknen des Spritzbelages werden die Pflanzen mit einer wäßrigen Sporensuspension von Phytophthora infestans inokuliert.

Die Pflanzen werden in einer Inkubationskabine mit 100 % relativer Luftfeuchtigkeit und ca. 20 °C aufgestellt.

3 Tage nach der Inokulation erfolgt die Auswertung.

Eine deutliche Überlegenheit in der Wirksamkeit gegenüber dem Stand der Technik zeigen in diesem Test z. B. die Verbindungen gemäß den Herstellungsbeispielen 2 und 17.

Beispiel D

Plasmopara-Test (Reben)/protektiv

Lösungsmittel : 4,7 Gewichtsteile Aceton
Emulgator : 0,3 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit bespritzt man junge Pflanzen mit der Wirkstoffzubereitung bis zur Tropfnässe. Nach Antrocknen des Spritzbelages werden die Pflanzen mit einer wäßrigen Sporensuspension von Plasmopara viticola inokuliert und verbleiben dann 1 Tag in einer Feuchtkammer bei 20 bis 22 °C und 100 % relativer Luftfeuchtigkeit. Anschließend werden die Pflanzen 5 Tage im Gewächshaus bei 22 °C und ca. 80 % Luftfeuchtigkeit aufgestellt. Die Pflanzen werden dann angefeuchtet und 1 Tag in eine Feuchtkammer gestellt.

7 Tage nach der Inokulation erfolgt die Auswertung.

Eine deutliche Überlegenheit in der Wirksamkeit gegenüber dem Stand der Technik zeigen in diesem Test z. B. die Verbindungen gemäß den Herstellungsbeispielen 1 und 23.

**Patentansprüche**

1. Thiolan-2,4-dion-3-carboxamide der allgemeinen Formel (I),

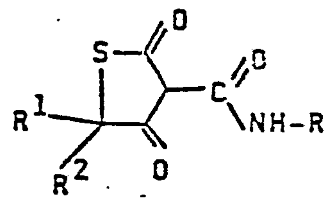

(I)

in welcher

R für geradkettiges oder verzweigtes Alkyl mit 1 bis 8 Kohlenstoffatomen, für Cycloalkyl mit 3 bis 7 Kohlenstoffatomen, für gegebenenfalls einfach oder mehrfach, gleich oder verschieden substituiertes Aralkyl mit 1 oder 2 Kohlenstoffatomen im Alkylteil und 6 bis 10 Kohlenstoffatomen im Arylteil, sowie für gegebenenfalls einfach oder mehrfach, gleich oder verschieden substituiertes Aryl mit 6 bis 10 Kohlenstoffatomen steht, wobei als Arylsubstituenten jeweils in Frage kommen : Halogen, Cyano, Nitro, geradkettiges oder verzweigtes Alkyl, mit 1 bis 16 Kohlenstoffatomen, geradkettiges oder verzweigtes Alkoxy und Alkylthio mit jeweils 1 bis 4 Kohlenstoffatomen, Halogenalkyl, Halogenalkoxy und Halogenalkylthio mit jeweils 1 oder 2 Kohlenstoffatomen und 1 bis 5 gleichen oder verschiedenen Halogenatomen, gegebenenfalls durch Halogen oder niederes Alkyl einfach oder mehrfach, gleich oder verschieden substituiertes zweifach verknüpftes Dioxyalkylen mit 1 oder 2 Kohlenstoffatomen im Alkylenrest, jeweils gegebenenfalls einfach oder mehrfach, gleich oder verschieden durch Halogen, niederes Alkyl und niederes Halogenalkyl substituiertes Phenyl oder Phenoxy, ferner Cycloalkyl mit 5 bis 7 Kohlenstoffatomen sowie Alkoxycarbonylalkyl und Alkoxycarbonylalkenyl mit jeweils 1 oder 2 Kohlenstoffatomen im Alkoxyteil und jeweils bis zu 2 Kohlenstoffatomen im Alkyl- bzw. Alkenylteil und

$R^1$ und $R^2$ unabhängig voneinander für Wasserstoff, für geradkettiges oder verzweigtes Alkyl mit 1 bis 6 Kohlenstoffatomen, für Cycloalkyl mit 3 bis 7 Kohlenstoffatomen, für Cycloalkylalkyl mit 3 bis 7 Kohlenstoffatomen im Cycloalkylteil und 1 oder 2 Kohlenstoffatomen im Alkylteil, für Phenylalkyl mit 1 oder 2 Kohlenstoffatomen im Alkylteil oder für Phenyl stehen.

2. Thiolan-2,4-dion-3-carboxamide der Formel (I) gemäß Anspruch 1, in welcher

R für geradkettiges oder verzweigtes Alkyl mit 1 bis 6 Kohlenstoffatomen, für Cyclopentyl, für Cyclohexyl oder für gegebenenfalls einfach bis dreifach, gleich oder verschieden substituiertes Phenyl, Benzyl oder Naphthyl steht, wobei als Substituenten jeweils in Frage kommen : Fluor, Chlor, Brom, Nitro, Cyano, geradkettiges oder verzweigtes Alkyl mit 1 bis 14 Kohlenstoffatomen, Methoxy, Ethoxy, Methylthio, Trifluormethyl, Trifluormethoxy, Trifluormethylthio, jeweils zweifach verknüpftes Dioxymethylen, Dioxyethylen oder Dioxytrifluorchlorethylen, jeweils gegebenenfalls einfach bis dreifach, gleich oder verschieden durch Methyl, Chlor oder Trifluormethyl substituiertes Phenyl oder Phenoxy, Cyclopentyl oder Cyclohexyl, 2-Methoxycarbonylvinyl und 2-Ethoxycarbonylvinyl und

$R^1$ und $R^2$ unabhängig voneinander für Wasserstoff, Methyl, Ethyl, n- und i-Propyl, n-, i-, s- und t-Butyl, Cyclohexyl, Cyclohexylmethyl, Benzyl oder Phenyl stehen.

3. Verfahren zur Herstellung von Thiolan-2,4-dion-3-carboxamiden der allgemeinen Formel (I),

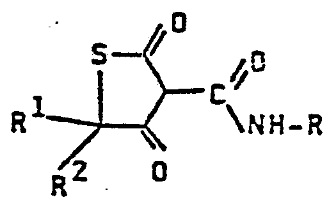

(I)

in welcher

R für geradkettiges oder verzweigtes Alkyl mit 1 bis 8 Kohlenstoffatomen, für Cycloalkyl mit 3 bis 7 Kohlenstoffatomen, für gegebenenfalls einfach oder mehrfach, gleich oder verschieden substituiertes Aralkyl mit 1 oder 2 Kohlenstoffatomen im Alkylteil und 6 bis 10 Kohlenstoffatomen im Arylteil, sowie für gegebenenfalls einfach oder mehrfach, gleich oder verschieden substituiertes Aryl mit 6 bis 10 Kohlenstoffatomen steht, wobei als Arylsubstituenten jeweils in Frage kommen : Halogen, Cyano, Nitro, geradkettiges oder verzweigtes Alkyl, mit 1 bis 16 Kohlenstoffatomen, geradkettiges oder verzweigtes Alkoxy und Alkylthio mit jeweils 1 bis 4 Kohlenstoffatomen, Halogenalkyl, Halogenalkoxy und Halogenalkylthio mit jeweils 1 oder 2 Kohlenstoffatomen und 1 bis 5 gleichen oder verschiedenen Halogenatomen, gegebenenfalls durch Halogen oder niederes Alkyl einfach oder mehrfach, gleich oder verschieden substituiertes zweifach verknüpftes Dioxyalkylen mit 1 oder 2 Kohlenstoffatomen im Alkylenrest, jeweils gegebenenfalls einfach oder mehrfach, gleich oder verschieden durch Halogen, niederes Alkyl und niederes Halogenalkyl substituiertes Phenyl oder Phenoxy, ferner Cycloalkyl mit 5 bis 7 Kohlenstoffatomen sowie Alkoxycarbonylalkyl und Alkoxycarbonylalkenyl mit jeweils 1 oder 2 Kohlenstoffatomen im Alkoxyteil und jeweils bis zu 2 Kohlenstoffatomen im Alkyl- bzw. Alkenylteil und

$R^1$ und $R^2$ unabhängig voneinander für Wasserstoff, für geradkettiges oder verzweigtes Alkyl mit 1 bis 6 Kohlenstoffatomen, für Cycloalkyl mit 3 bis 7 Kohlenstoffatomen, für Cycloalkylalkyl mit 3 bis 7

Kohlenstoffatomen im Cycloalkylteil und 1 oder 2 Kohlenstoffatomen im Alkylteil, für Phenylalkyl mit 1 oder 2 Kohlenstoffatomen im Alkylteil oder für Phenyl stehen,
dadurch gekennzeichnet, daß man Thiolan-2,4-dion-3-carbonsäureester der Formel (II),

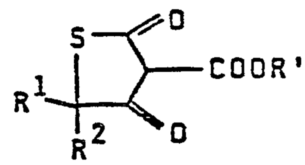

(II)

in welcher

$R^1$ und $R^2$ die oben angegebenen Bedeutungen haben und
$R'$ für Alkyl steht,

mit Aminen der Formel (III),

$$H_2N—R \quad (III)$$

in welcher R die oben angegebenen Bedeutungen hat, gegebenenfalls in Gegenwart eines Verdünnungsmittels umsetzt.

4. Schädlingsbekämpfungsmittel, gekennzeichnet durch einen Gehalt an mindestens einem Thiolan-2,4-dion-3-carboxamid der Formel (I) gemäß den Ansprüchen 1 und 3.

5. Mikrobizide Mittel, gekennzeichnet durch einen Gehalt an mindestens einem Thiolan-2,4-dion-3-carboxamid der Formel (I) gemäß den Ansprüchen 1 und 3.

6. Verfahren zur Bekämpfung von Schädlingen, dadurch gekennzeichnet, daß man Thiolan-2,4-dion-3-carboxamide der Formel (I) gemäß den Ansprüchen 1 und 3 auf Schädlinge und/oder ihren Lebensraum einwirken läßt.

7. Verwendung von Thiolan-2,4-dion-3-carboxamiden der Formel (I) gemäß den Ansprüchen 1 und 3 zur Bekämpfung von Schädlingen.

8. Verwendung von Thiolan-2,4-dion-3-carboxamiden der Formel (I) gemäß den Ansprüchen 1 und 3 zur Bekämpfung von Pilzen und Bakterien.

9. Verfahren zur Herstellung von Schädlingsbekämpfungsmitteln, dadurch gekennzeichnet, daß man Thiolan-2,4-dion-3-carboxamide der Formel (I) gemäß den Ansprüchen 1 und 3 mit Streckmitteln und/oder oberflächenaktiven Mitteln vermischt.

**Claims**

1. Thiolane-2,4-dione-3-carboxamides of the general formula (I)

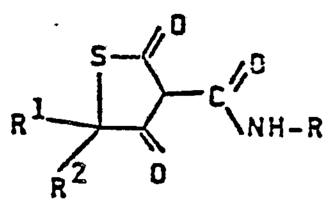

(I)

in which

R represents straight-chain or branched alkyl with 1 to 8 carbon atoms, cycloalkyl with 3 to 7 carbon atoms, aralkyl which has 1 or 2 carbon atoms in the alkyl part and 6 to 10 carbon atoms in the aryl part and is optionally mono- or polysubstituted by identical or different substituents, and aryl which has 6 to 10 carbon atoms and is optionally mono- or polysubstituted by identical or different substituents, possible aryl substituents in each case being : halogen, cyano, nitro, straight-chain or branched alkyl, with 1 to 16 carbon atoms, straight-chain or branched alkoxy and alkylthio with in each case 1 to 4 carbon atoms, halogenoalkyl, halogenoalkoxy and halogenoalkylthio with in each case 1 or 2 carbon atoms and 1 to 5 identical or different halogen atoms, dioxyalkylene which has 1 or 2 carbon atoms in the alkylene radical, is linked in two positions and is optionally mono- or polysubstituted by identical or different substituents from amongst halogen and lower alkyl, phenyl or phenoxy which are in each case optionally mono- or polysubstituted by identical or different substituents from amongst halogen, lower alkyl and lower halogenoalkyl, as well as cycloalkyl with 5 to 7 carbon atoms and alkoxycarbonylalkyl and alkoxycarbonylalkenyl with in each case 1 or 2 carbon atoms in the alkoxy part and in each case up to 2 carbon atoms in the alkyl or alkenyl part and

$R^1$ and $R^2$ independently on one another represent hydrogen, straight-chain or branched alkyl with 1 to 6 carbon atoms, cycloalkyl with 3 to 7 carbon atoms, cycloalkylalkyl with 3 to 7 carbon atoms in the

cycloalkyl part and 1 or 2 carbon atoms in the alkyl part, phenylalkyl with 1 or 2 carbon atoms in the alkyl part or phenyl.

2. Thiolane-2,4-dione-3-carboxamides of the formula (I) according to claim 1, in which

R represents straight-chain or branched alkyl with 1 to 6 carbon atoms, cyclopentyl, cyclohexyl, or phenyl, benzyl or naphthyl which are optionally mono- to trisubstituted by identical or different substituents, possible substituents in each case being : fluorine, chlorine, bromine, nitro, cyano, straight-chain or branched alkyl with 1 to 14 carbon atoms, methoxy, ethoxy, methylthio, trifluoromethyl, trifluoromethoxy, trifluoromethylthio, dioxymethylene, dioxyethylene or dioxytrifluorochloroethylene, each of which is linked in two positions, phenyl or phenoxy, each of which is optionally mono- to trisubstituted by identical or different substituents from amongst methyl, chlorine and trifluoromethyl, cyclopentyl or cyclohexyl, 2-methoxycarbonylvinyl and 2-ethoxycarbonylvinyl and

$R^1$ and $R^2$ independently of one another represent hydrogen, methyl, ethyl, n- and i-propyl, n-, i-, s- and t-butyl, cyclohexyl, cyclohexylmethyl, benzyl or phenyl.

3. Process for the preparation of thiolane-2,4-dione-3-carboxamides of the general formula (I)

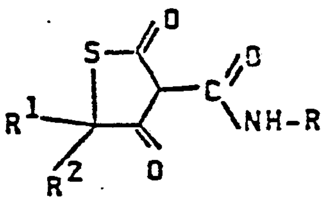

(I)

in which

R represents straight-chain or branched alkyl with 1 to 8 carbon atoms, cycloalkyl with 3 to 7 carbon atoms, aralkyl which has 1 or 2 carbon atoms in the alkyl part and 6 to 10 carbon atoms in the aryl part and is optionally mono- or polysubstituted by identical or different substituents, and aryl which has 6 to 10 carbon atoms and is optionally mono- or polysubstituted by identical or different substituents, possible aryl substituents in each case being : halogen, cyano, nitro, straight-chain or branched alkyl, with 1 to 16 carbon atoms, straight-chain or branched alkoxy and alkylthio with in each case 1 to 4 carbon atoms, halogenoalkyl, halogenoalkoxy and halogenoalkylthio with in each case 1 or 2 carbon atoms and 1 to 5 identical or different halogen atoms, dioxyalkylene which has 1 or 2 carbon atoms in the alkylene radical, is linked in two positions and is optionally mono- or polysubstituted by identical or different substituents from amongst halogen and lower alkyl, phenyl or phenoxy which are in each case optionally mono- or polysubstituted by identical or different substituents from amongst halogen, lower alkyl and lower halogenoalkyl, as well as cycloalkyl with 5 to 7 carbon atoms and alkoxycarbonylalkyl and alkoxycarbonylalkenyl with in each case 1 or 2 carbon atoms in the alkoxy part and in each case up to 2 carbon atoms in the alkyl or alkenyl part and

$R^1$ and $R^2$ independently on one another represent hydrogen, straight-chain or branched alkyl with 1 to 6 carbon atoms, cycloalkyl with 3 to 7 carbon atoms, cycloalkylalkyl with 3 to 7 carbon atoms in the cycloalkyl part and 1 or 2 carbon atoms in the alkyl part, phenylalkyl with 1 or 2 carbon atoms in the alkyl part or phenyl,

characterised in that thiolane-2,4-dione-3-carboxylic acid esters of the formula (II)

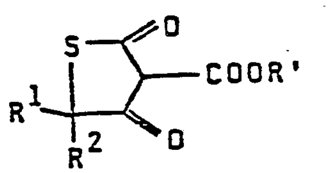

(II)

in which

$R^1$ and $R^2$ have the abovementioned meanings and

$R^1$ represents alkyl,

are reacted with amines of the formula (III),

$$H_2N—R \quad (III)$$

in which R has the abovementioned meanings, if appropriate in the presence of a diluent.

4. Agents for combating pests, characterised in that they contain at least one thiolane-2,4-dione-3-carboxamide of the formula (I) according to claims 1 and 3.

5. Microbicidal agents, characterised in that they contain at least one thiolane-2,4-dione-3-carboxamide of the formula (I) according to claims 1 and 3.

6. Process for combating pests, characterised in that thiolane-2,4-dione-3-carboxamides of the formula (I) according to claims 1 and 3 are allowed to act on pests and/or their environment.

7. Use of thiolane-2,4-dione-3-carboxamides of the formula (I) according to claims 1 and 3 for combating pests.

8. Use of thiolane-2,4-dione-3-carboxamides of the formula (I) according to claims 1 and 3 for combating fungi and bacteria.

9. Process for the preparation of agents for combating pests, characterised in that thiolane-2,4-dione-3-carboxamides of the formula (I) according to claims 1 and 3 are mixed with extenders and/or surface-active agents.

## Revendications

1. Thiolane-2,4-dione-3-carboxamides de formule générale (I)

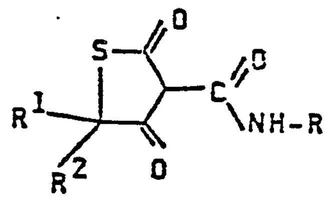

(I)

dans laquelle

R représente un groupe alkyle à chaîne droite ou ramifié ayant 1 à 8 atomes de carbone, un groupe cycloalkyle de 3 à 7 atomes de carbone, un groupe aralkyle ayant 1 ou 2 atomes de carbone dans la partie alkyle et 6 à 10 atomes de carbone dans la partie aryle, portant éventuellement un ou plusieurs substituants identiques ou différents, ainsi qu'un groupe aryle de 6 à 10 atomes de carbone, portant éventuellement un ou plusieurs substituants identiques ou différents, et on considère alors dans chaque cas comme substituants des groupes aryle : un halogène, un groupe cyano, un groupe nitro, un groupe alkyle à chaîne droite ou ramifié ayant 1 à 16 atomes de carbone, un groupe alkoxy et un groupe alkylthio à chaîne droite ou ramifiés ayant chacun 1 à 4 atomes de carbone, des groupes halogénalkyle, halogénalkoxy et halogénalkylthio ayant chacun 1 ou 2 atomes de carbone et 1 à 5 atomes d'halogènes identiques ou différents, un groupe dioxyalkylène à liaison double ayant 1 ou 2 atomes de carbone dans le reste alkylène, portant éventuellement un ou plusieurs substituants halogéno ou alkyle inférieur identiques ou différents, un groupe phényle ou phénoxy portant éventuellement un ou plusieurs substituants identiques ou différents tels qu'halogéno, alkyle inférieur et halogénalkyle inférieur, en outre, un groupe cycloalkyle de 5 à 7 atomes de carbone de même qu'un groupe alkoxycarbonylalkyle et un groupe alkoxycarbonylalcényle ayant chacun 1 ou 2 atomes de carbone dans la partie alkoxy et chacun jusqu'à 2 atomes de carbone dans la partie alkyle ou alcényle, et

$R^1$ et $R^2$ représentent, indépendamment l'un de l'autre, l'hydrogène, un groupe alkyle à chaîne droite ou ramifié ayant 1 à 6 atomes de carbone, un groupe cycloalkyle de 3 à 7 atomes de carbone, un groupe cycloalkylalkyle ayant 3 à 7 atomes de carbone dans la partie cycloalkyle et 1 ou 2 atomes de carbone dans la partie alkyle, un groupe phénylalkyle ayant 1 ou 2 atomes de carbone dans la partie alkyle, ou le groupe phényle.

2. Thiolane-2,4-dione-3-carboxamide de formule (I) suivant la revendication 1, dans laquelle

R est un groupe alkyle à chaîne droite ou ramifié ayant 1 à 6 atomes de carbone, le groupe cyclopentyle, le groupe cyclohexyle ou un groupe phényle, benzyle ou naphtyle, portant éventuellement 1 à 3 substituants identiques ou différents, et on considère alors dans chaque cas comme substituants : le fluor, le chlore, le brome, un groupe nitro, cyano, alkyle de 1 à 14 atomes de carbone à chaîne droite ou ramifié, méthoxy, éthoxy, méthylthio, trifluorométhyle, trifluorométhoxy, trifluorométhylthio, un groupe dioxyméthylène, dioxyéthylène ou dioxytrifluorochloréthylène à liaison double dans chaque cas, un groupe phényle ou phénoxy portant chacun le cas échéant 1 à 3 substituants méthyle, chloro ou trifluorométhyle identiques ou différents, le groupe cyclopentyle ou le groupe cyclohexyle, le groupe 2-méthoxycarbonylvinyle et le groupe 2-éthoxycarbonylvinyle, et

$R^1$ et $R^2$ représentent indépendamment l'un de l'autre l'hydrogène, un groupe méthyle, éthyle, n-propyle, isopropyle, n-butyle, isobutyle, sec.-butyle, tertio-butyle, cyclohexyle, cyclohexyl-méthyle, benzyle ou phényle.

3. Procédé de préparation de thiolane-2,4-dione-3-carboxamides de formule générale (I)

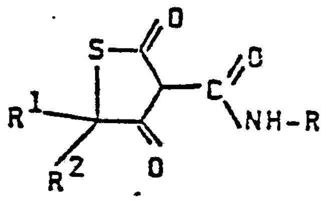

(I)

dans laquelle

R représente un groupe alkyle à chaîne droite ou ramifié ayant 1 à 8 atomes de carbone, un groupe cycloalkyle de 3 à 7 atomes de carbone, un groupe aralkyle ayant 1 ou 2 atomes de carbone dans la partie alkyle et 6 à 10 atomes de carbone dans la partie aryle, portant éventuellement un ou plusieurs substituants identiques ou différents, ainsi qu'un groupe aryle de 6 à 10 atomes de carbone, portant éventuellement un ou plusieurs substituants identiques ou différents, et on considère alors dans chaque cas comme substituants des groupes aryle : un halogène, un groupe cyano, un groupe nitro, un groupe alkyle à chaîne droite ou ramifié ayant 1 à 16 atomes de carbone, un groupe alkoxy et un groupe alkylthio à chaîne droite ou ramifiés ayant chacun 1 à 4 atomes de carbone, des groupes halogénalkyle, halogénalkoxy et halogénalkylthio ayant chacun 1 ou 2 atomes de carbone et 1 à 5 atomes d'halogènes identiques ou différents, un groupe dioxyalkylène à liaison double ayant 1 ou 2 atomes de carbone dans le reste alkylène, portant éventuellement un ou plusieurs substituants halogéno ou alkyle inférieur identiques ou différents, un groupe phényle ou phénoxy portant éventuellement un ou plusieurs substituants identiques ou différents tels qu'halogéno, alkyle inférieur et halogénalkyle inférieur, en outre, un groupe cycloalkyle de 5 à 7 atomes de carbone de même qu'un groupe alkoxycarbonylalkyle et un groupe alkoxycarbonylalcényle ayant chacun 1 ou 2 atomes de carbone dans la partie alkoxy et chacun jusqu'à 2 atomes de carbone dans la partie alkyle ou alcényle, et

$R^1$ et $R^2$ représentent, indépendamment l'un de l'autre, l'hydrogène, un groupe alkyle à chaîne droite ou ramifié ayant 1 à 6 atomes de carbone, un groupe cycloalkyle de 3 à 7 atomes de carbone, un groupe cycloalkylalkyle ayant 3 à 7 atomes de carbone dans la partie cycloalkyle et 1 ou 2 atomes de carbone dans la partie alkyle, un groupe phénylalkyle ayant 1 ou 2 atomes de carbone dans la partie alkyle, ou le groupe phényle,

caractérisé en ce qu'on fait réagir des esters d'acides thiolane-2,4-dione-3-carboxyliques de formule (II)

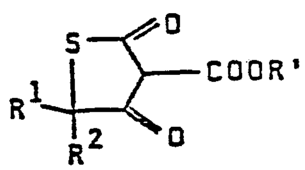

(II)

dans laquelle

$R^1$ et $R^2$ ont les définitions indiquées ci-dessus, et

R' est un groupe alkyle,

avec des amines de formule (III)

$$H_2N{-}R \quad (III)$$

dans laquelle R a les définitions indiquées ci-dessus, éventuellement en présence d'un diluant.

4. Compositions pesticides, caractérisées par une teneur en au moins un thiolane-2,4-dione-3-carboxamide de formule (I) suivant les revendications 1 et 3.

5. Compositions microbicides, caractérisées par une teneur en au moins un thiolane-2,4-dione-3-carboxamide de formule (I) suivant les revendications 1 et 3.

6. Procédé pour combattre des parasites, caractérisé en ce qu'on fait agir des thiolane-2,4-dione-3-carboxamide de formule (I) suivant les revendications 1 et 3 sur des parasites et/ou sur leur habitat.

7. Utilisation de thiolane-2,4-dione-3-carboxamides de formule (I) suivant les revendications 1 et 3 pour combattre des parasites.

8. Utilisation de thiolane-2,4-dione-3-carboxamides de formule (I) suivant les revendications 1 et 3 pour combattre des champignons et des bactéries.

9. Procédé de préparation de compositions pesticides, caractérisé en ce qu'on mélange des thiolanes 2,4-dione-3-carboxamide de formule (I) suivant les revendications 1 et 3 avec des diluants et/ou des agents tensio-actifs.